# EUROPÄISCHE PATENTANMELDUNG

(11) **EP 0 635 469 A1**
(43) Veröffentlichungstag der Anmeldung: **25.01.1995**
(21) Anmeldenummer: 94110964.7
(22) Anmeldetag: 14.07.1994
(51) Int. Cl.: C07C 25/24, C07C 17/263, C07C 17/266

(54) **2,4,5-Trihalogenstilbene, Verfahren zu ihrer Herstellung und ihre Verwendung**

(30) Priorität: 23.07.1993 DE 4324751
(71) Anmelder: HOECHST AKTIENGESELLSCHAFT, D-65929 Frankfurt am Main (DE)
(72) Erfinder: Beller, Matthias, Dr., D-65527 Niedernhausen (DE); Fischer, Hartmut, D-65719 Hofheim (DE); Weisse, Laurent, Dr., D-61440 Oberursel (DE); Forstinger, Klaus, Dr., D-65451 Kelsterbach (DE); Pfirmann, Ralf, Dr., D-64347 Griesheim (DE); Strutz, Heinz, Dr., D-61250 Usingen (DE)

(57) **Zusammenfassung**

Verbindungen der Formel I 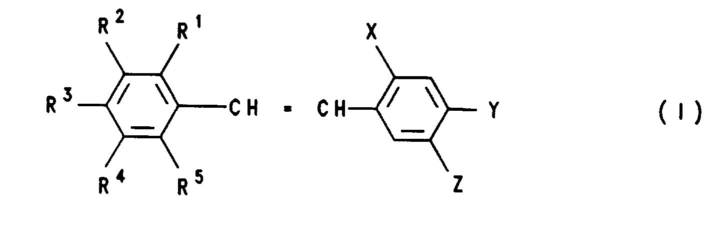
worin R¹ bis R⁵ unabhängig voneinander Wasserstoff, Alkyl(C₁-C₁₂), Fluor, Chlor, Brom, -OH, -CN, -CHO, -OAlkyl(C₁-C₈), -OPhenyl, -OCOAlkyl(C₁-C₈), -OCOPhenyl, -NH₂, -NHAlkyl(C₁-C₈), -NHPhenyl, -N(Alkyl)₂ (C₁-C₈), -NPhenylAlkyl(C₁-C₈), -N(Phenyl)₂, -NHCOAlkyl(C₁-C₈), -NHCOPhenyl, -NO₂, CN, Alkenyl, Alkinyl, -CF₃ und X, Y, Z ein Halogenatom bedeuten.

Weiterhin ein Verfahren zur Herstellung der Verbindungen der Formel (I), indem man eine Verbindung der Formel (II) 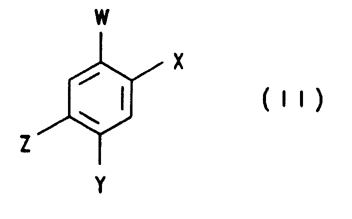
worin X, Y, Z die bereits genannte Bedeutung haben und W ein Iod-, Brom-, Chloratom oder eine Diazoniumgruppe N₂A bedeutet, worin A ein Anion einer Säure mit einem pKₐ-Wert < 7 ist, mit einer Verbindung der allgemeinen Formel (III) 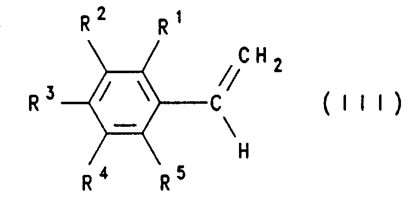
in welcher R¹ bis R⁵ die genannte Bedeutung haben, in Gegenwart katalytischer Mengen eines Palladiumkatalysators, gegebenenfalls in einem Lösungsmittel, gegebenenfalls in Gegenwart einer Base und/oder stabilisierender Liganden und/oder quartärer Ammonium- oder Phosphoniumsalze umsetzt.

## Beschreibung

Substituierte Stilbene haben vielfältige technische Bedeutung. Sie werden als Aufheller eingesetzt und sind wertvolle Zwischenprodukte für die Herstellung von Pflanzenschutzmitteln, Pharmazeutika und technischen Produkten, zum Beispiel optischen Aufhellern.

Die Herstellung von substituierten Stilbenderivaten erfolgt üblicherweise durch oxidative Dimerisierung von Methylaromaten, Dehydratisierung von 1,2-Diarylethanolen, Kondensation von Methylaromaten mit Arylaldehyden oder Aryliminen (Anil-Synthese) und Wittig-Reaktion von Benzylhalogeniden mit Arylaldehyden. Eine Übersicht der verschiedenen Stilbensynthesen findet man beispielsweise in K. B. Becker, Synthesis, 1983, Seite 341-368. Bei der Heck-Reaktion von Arylhalogeniden mit Styrolen zu Stilbenen sind überwiegend Aryliodide oder Arylbromide eingesetzt worden (A. Spencer, J. Organomet, Chem., 258 (1983) 101; W. Heitz, Makromol. Chem., 198 (1988) 119; EP 104 296).

Wegen der allgemeinen Bedeutung und der vielseitigen Verwendbarkeit dieser Stoffklasse stellt es eine lohnende Aufgabe dar, neue Verbindungen aus dieser Gruppe von Stoffen bereitzustellen, um das Spektrum ihrer Anwendungsmöglichkeiten nicht nur zu ergänzen, sondern auch durch eine Nuancierung stofflicher Eigenschaften zu bereichern und zu erweitern. Zudem eröffnen sich mittels dieser neuen Verbindungen zusätzlich Wege zur Herstellung von Folgeprodukten, deren Bedeutung voranstehend bereits erwähnt wurde.

Diese Aufgabe wird gelöst durch Verbindungen der Formel (I) 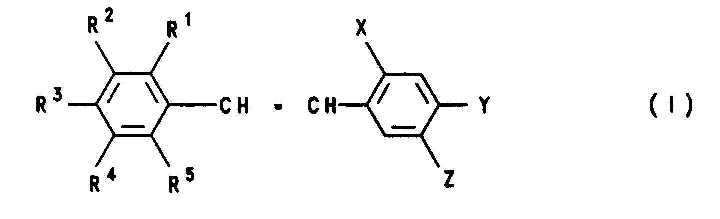
dadurch gekennzeichnet, daß R¹ bis R⁵ unabhängig voneinander Wasserstoff, Alkyl(C₁-C₁₂), Fluor, Chlor, Brom, -OH, -CN, -CHO, -OAlkyl(C₁-C₈), -OPhenyl, -OCOAlkyl(C₁-C₈), -OCOPhenyl, -NH₂, -NHAlkyl(C₁-C₈), -NHPhenyl, -N(Alkyl)₂ (C₁-C₈), -NPhenylAlkyl(C₁-C₈), -N(Phenyl)₂, -NHCOAlkyl(C₁-C₈), -NHCOPhenyl, -NO₂, CN, Alkenyl, Alkinyl, -CF₃ und X, Y, Z ein Halogenatom bedeuten.

Als Reste R¹ bis R⁵ kommen beispielsweise Methyl-, Ethyl-, n-Propyl-, Isopropyl-, n-Butyl-, i-Butyl-, tert.-Butyl-, n-Pentyl-, i-Pentyl-, n-Hexyl-, n-Heptyl-, n-Octyl-, 2-Ethylhexyl-, 2-Ethyldecyl-, n-Decyl-, n-Dodecyl-, ferner Methoxy-, Ethoxy-, Propoxy-, Isopropoxy-, Butoxy-, Pentyloxy-, Hexyloxy-, Octyloxy-, 2-Ethylhexyloxy-, Decyloxy-, Dodecyloxy-, ferner Carbonsäure-, Carbonsäuremethylester-, Carbonsäureethylester-, Carbonsäurepropylester-, Carbonsäurebutylester-, Carbonsäurepentylester-, Carbonsäurehexylester-, Carbonsäure-2-ethylhexylester-, Carbonsäurephenylester-, Formyl-, Carbonsäureamid-, Carbonsäuredimethylamid-, Carbonsäurediethylamid-, Carbonsäuremethylamid-, Carbonsäureethylamid, ferner N,N-Dimethylamino-, N,N-Diethylamino-, N-Methylamino-, N-Ethylamino-, N,N-Methylethylamino-, N,N-Dipropylamino-, N,N-Dibutylamino-, N-Morpholino-, schließlich Acetoxy-, Propionyloxy- und Butyryloxygruppen, in Betracht.

Eine gewisse Bedeutung kommt den Verbindungen der Formel (I) zu, worin R¹ bis R⁵ für Wasserstoff, Alkyl(C₁-C₁₂) und Halogen steht, insbesondere Wasserstoff, Methyl, Ethyl, Fluor oder Chlor.

Interessant sind auch die Verbindungen der Formel (I) worin X, Y, Z für Fluor, Chlor und/oder Brom, insbesondere für Fluor und/oder Chlor steht.

Von besonderem Interesse sind 2,4-Dichlor-5-fluor-stilben, 2,4,5-Trifluorstilben, 2-Chlor-4,5-difluorstilben, 2,4-Dichlor-5-fluor-4'-methylstilben, 2,4-Dichlor-5-fluor-2'-methylstilben, 2,4-Dichlor-5-fluor-3'-methylstilben, 2,4,4'-Trichlor-5-fluorstilben.

Die vorliegende Erfindung betrifft ferner ein Verfahren zur Herstellung der erfindungsgemäßen Verbindungen.

Die neuen Stilbenderivate der Formel (I) lassen sich in vorteilhafter Weise herstellen, indem man eine aromatische Halogenverbindungen oder ein aromatisches Diazoniumsalz der allgemeinen Formel (II) 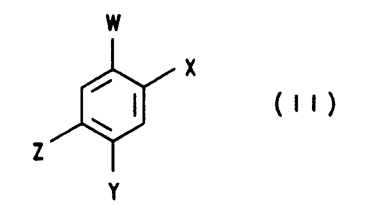
worin X, Y, Z die bereits genannte Bedeutung haben und W ein Iod-, Brom-, Chloratom oder eine Diazoniumgruppe N₂A bedeutet, worin A ein Anion einer Säure mit einem pKₐ-Wert < 7 ist, mit einer Verbindung der allgemeinen Formel (III) 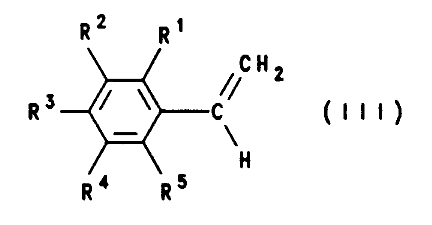
in welcher R¹ bis R⁵ die genannte Bedeutung haben, in Gegenwart katalytischer Mengen eines Palladiumkatalysators, gegebenenfalls in einem Lösungsmittel, gegebenenfalls in Gegenwart einer Base und/oder stabilisierender Liganden und/oder quartärer Ammoniumsalze umsetzt.

Der Palladiumkatalysator kann sowohl homogen als auch heterogen eingesetzt werden. Bei homogenen Palladiumkatalysatoren ist es im allgemeinen von Vorteil stabilisierende Liganden wie Phosphine, Phosphite, Nitrile oder Amine zuzusetzen, die sowohl monofunktionell als auch chelatisierend sein können. Zur Stabilisierung des Katalysators kann es außerdem von Vorteil sein Phasentransferreagenzien wie quartäre Ammonium- oder Phosphoniumsalze beispielsweise Tetra-n-butylammoniumbromid, Tetramethylammoniumchlorid, Tetraphenylammoniumchlorid, Hexadecyltrimethylammoniumbromid und/oder Tetraphenylphosphoniumbromid zuzusetzen.

Geeignete Stabilisatoren für die homogenen Palladiumkatalysatoren sind insbesondere Triaryl- und Trialkylphosphine, beispielsweise Triphenylphosphin, Tricyclohexylphosphin, Tri-n-butylphosphin, Tri-o-tolylphosphin, Bisdiphenylphosphinoalkane, aber auch Elektronendonorverbindungen wie Acetonitril, Benzonitril, Bispyridin, Bisdimethylaminoalkalane und Triethylphosphit.

Geeignete homogene Palladiumverbindungen sind Palladium(II)salze wie beispielsweise Palladiumacetat, Palladiumdichlorid, Palladiumdibromid, Natriumtetrachloropalladat, Bistriphenylphosphinpalladiumdichlorid, Bisbenzonitrilpalladiumdichlorid, Palladiumsulfat, aber auch Palladium(O)komplexe wie Tetrakistriphenylphosphinpalladium und Palladiumbisbenzylidenacetonkomplexe. Sie können allein oder in Form beliebiger Gemische verwendet werden. Die Palladiumphosphinkomplexe werden bevorzugt in situ erzeugt.

Werden Diazoniumsalze eingesetzt, verwendet man die homogenen Palladiumkatalysatoren vorteilhaft ohne stabilisierende Liganden. Bewährt haben sich in diesem Fall Palladiumacetat, Palladiumdichlorid und Palladiumdibenzylidenacetonkomplexe als Katalysator.

Bei der Verwendung heterogener Palladiumträgerkatalysatoren wird zweckmäßigerweise ein Trägermaterial wie beispielsweise Aktivkohle, Calciumcarbonat, Bariumsulfat, Bimsstein, Tonerde, Kieselgur, Kieselgel, Graphit, Magnesiumoxid und/oder Aluminiumoxid angewendet. Bevorzugt wird Palladium auf Aktivkohle und/oder Aluminiumoxid und/oder Kieselgel zur Anwendung gebracht.

Bei den heterogenen Palladiumkatalysatoren ist es zweckmäßig, einen Palladiumkatalysator anzuwenden, der etwa 0,5 - 20 Gew.-% insbesondere 1 - 10 Gew.-% Palladium, bezogen auf das Trägermaterial, enthält.

Hinsichtlich des Mengenverhältnisses Palladium zu Halogenaromat oder Diazoniumverbindung ist es zweckmäßig, etwa 0,001 bis etwa 20 mol %, insbesondere etwa 0,05 bis etwa 5 mol % Palladium, bezogen auf den Halogenaromaten oder die Diazoniumverbindung, anzuwenden.

Was das Mengenverhältnis der Reaktanden anbelangt, hat es sich häufig bewährt 1 mol Halogenaromat oder Diazoniumverbindung der allgemeinen Formel II mit etwa 0,5 - 10 mol, insbesondere mit 1 - 5 mol Styrol der Formel (III) zur Umsetzung zu bringen.

Das Verfahren wird vorzugsweise in einem Lösungsmittel, bei den Halogenaromaten in der Regel in einem organischen Lösungsmittel wie einem dipolar aprotischen Lösungsmittel wie Dimethylacetamid, Dimethylformamid, N-Methylpyrrolidon, Dimethylsulfoxid oder Ethern, beispielsweise Diglyme oder Tetraglyme durchgeführt, es kann aber auch bei bestimmten Halogenaromaten ohne Lösungsmittel durchgeführt werden.

Besonders bevorzugt sind als Lösungsmittel Dimethylacetamid, Dimethylformamid und N-Methylpyrrolidon.

Bei der Reaktion der Diazoniumsalze können neben den genannten dipolar aprotischen Lösungsmitteln auch Alkohole, wie Methanol, Ethanol, Isopropanol und Ethylenglykol, und Ether, wie Tetrahydrofuran und Dioxan, und Nitrile wie Acetonitril, aber auch in bestimmten Fällen Wasser, verwendet werden.

Unter Umständen ist es vorteilhaft, die Reaktionen in einem Mehrphasensystem beispielsweise bestehend aus einer organischen Phase, einer wäßrigen Phase und einem Trägerkatalysator durchzuführen.

Die Aryldiazoniumverbindungen lassen sich nach gängigen, dem Fachmann bekannten Methoden herstellen (Houben-Weyl, Methoden der organischen Chemie, X/3, Seiten 3 - 214). Beim Einsatz der Diazoniumsalze empfiehlt es sich, das Aryldiazoniumsalz zu isolieren und in reiner Form einzusetzen. Es ist aber auch möglich, das in Lösung hergestellte Diazoniumsalz mittels Palladiumkatalysatoren direkt zu den entsprechenden Stilbenen umzusetzen.

Bei der Reaktion der 2,4,5-Trihalogenarylhalogenide und gegebenenfalls bei der Umsetzung der Aryldiazoniumsalze ist es von Vorteil, die Reaktion in Gegenwart von Basen durchzuführen.

Geeignete Basen sind offenkettige oder cyclische Amine, wie Diethylamin, Triethylamin, Tri-n-butylamin, Tripropylamin, Benzylamin, Pyrrolidin, Piperidin, Pyridin, Dimethylpyridin, Diazabicyclooctan, Diazabicycloundecan, Arylamine, Alkali- und/oder Erdalkalisalze von aliphatischen und/oder aromatischen Carbonsäuren wie Natriumacetat, Natriumpropionat, Kaliumacetat sowie Alkali-und/oder Erdalkalisalze anorganischer Säuren, die einen pKa-Wert zwischen 2 und 14 besitzen, wie Natriumcarbonat, Kaliumcarbonat, Natriumhydrogencarbonat, Lithiumhydrogencarbonat, Kaliumhydroxid und Natriumhydroxid.

Die nachstehenden Beispiele dienen zur Erläuterung des erfindungsgemäßen Verfahrens, ohne es darauf zu beschränken.

### Beispiel 1

### 2,4-Dichlor-5-fluorstilben

50 g (0,205 mol) 1-Brom-2,4-dichlor-5-fluorbenzol, 42 g (0,410 mol) Styrol, 0,45 g (2,05 mmol) Palladiumacetat, 1,08 g (4,10 mmol) Triphenylphosphin, 0,82 g (2,05 mmol) Bisdiphenylphosphinoethan und 20,0 g (0,246 mol) Natriumacetat werden unter Schutzgas in 100 ml Dimethylacetamid gelöst und 18 Stunden unter Rückfluß gekocht. Nach Abkühlen der Lösung wird die Mischung mit 300 ml Dichlormethan verdünnt und zweimal mit 200 ml Wasser extrahiert. Die organische Phase wird im Vakuum eingeengt. Aus der verbleibenden zähen Lösung kristallisieren nach 1 - 2 Tagen 19 g (35 % Ausbeute) des gewünschten Produktes mit 97 % Reinheit. Durch Umkristallisation der Mutterlauge erhält man weitere 24 g des Produktes mit einer Reinheit > 95 %.
Ausbeute: 79 % 2,4-Dichlor-5-fluorstilben
¹H-NMR: 7,05 (d, J = 16 Hz, 1 H, C-H, olefinisch), 7,29 - 7,57 (m, 8 H, C-H, olefinisch, aromatisch)
¹⁹F-NMR (94 MHz): 117,8 (C-F)
MS (EI, 70 eV): 266 (100 %) M⁺

### Beispiel 2

### 2,4-Dichlor-5-fluorstilben

3,0 g (10,8 mmol) 2,4-Dichlor-5-fluorphenyldiazoniumtetrafluoroborat werden zu einer 60 - 70°C warmen Lösung von 122 mg Palladiumacetat, 13 ml Styrol und 30 ml Chloroform portionsweise gegeben. Anschließend wird zwei Stunden bei dieser Temperatur nachgerührt. Die Mischung wird im Vakuum eingeengt und chromatographiert (Elutionsmittel: Petrolether/Essigester 20 : 1, Träger SiO₂). Man erhält ein Gemisch von Styrol und Produkt, aus dem 2,4-Dichlor-5-fluorstilben langsam auskristallisiert.
Ausbeute: 83 % 2,4-Dichlor-5-fluorstilben

### Beispiel 3

### 2,4-Dichlor-5-fluorstilben

20 g (82 mmol) 1-Brom-2,4-dichlor-5-fluorbenzol, 18,7 ml Styrol, 1,74 g Palladium auf Aktivkohle (5 %ig) und 6,08 g Natriumcarbonat werden in 64 ml Dimethylacetamid gelöst. Anschließend wird 18 Stunden am Rückfluß gekocht und nach Abkühlen und Abfiltrieren des Katalysators wie in Beispiel 1 beschrieben aufgearbeitet.
Ausbeute: 70 % 2,4-Dichlor-5-fluorstilben.

### Beispiel 4

### 2,4,5-Trifuorstilben

3,0 g (14,4 mmol) 2,4,5-Trifluorbrombenzol werden mit 2,96 g (28,5 mmol) Styrol, 139 mg (0,14 mmol) Palladium auf Aktivkohle (5 %ig), 2 mg tert. Butylbrenzkatechin und 0,82 g (7,7 mmol) Natriumcarbonat in 10 ml Dimethylacetamid 18 Stunden bei 170°C am Rückfluß gekocht. Nach Abkühlen auf Raumtemperatur wird der Katalysator abfiltiert, die organische Phase mit Dichlormethan verdünnt, dreimal mit Wasser ausgeschüttelt und chromatographiert (Elutionsmittel: Petrolether/Essigester 20 : 1, Träger SiO₂).
Ausbeute: 72 % 2,4,5-Trifluorstilben
¹H-NMR: 6,90 - 7,54 (m, 9 H, C-H, olefinisch, aromatisch)
¹⁹F-NMR (94 MHz): 109,08 (C-F), 133,8 (C-F), 140,5 (C-F)
MS (EI, 70 eV): 234 (100 %) M⁺

### Beispiel 5

### 2,4,5-Trifuorstilben

3,0 g (14,4 mmol) 2,4,5-Trifluorbrombenzol werden mit 2,96 g (28,5 mmol) Styrol, 31 mg (0,14 mmol) Palladiumacetat, 75 mg (0,28 mmol) Triphenylphosphin, 57 mg (0,14 mmol) Bisdiphenylphosphinoethan, 2 mg tert. Butylbrenzkatechin und 1,40 g (17,0 mmol) Natriumacetat in 6 ml Dimethylacetamid 18 Stunden bei 150°C gerührt. Nach Abkühlen auf Raumtemperatur wird die organische Phase mit Dichlormethan verdünnt, dreimal mit Wasser ausgeschüttelt und chromatographiert (Elutionsmittel: Petrolether/Essigester 20 : 1, Träger SiO₂).
Ausbeute: 68 % 2,4,5-Trifuorstilben

### Beispiel 6

### 2,4-Dichlor-5-fluor-4'-methylstilben

16 g 2,4-Dichlor-5-fluoranilin werden mit 160 ml Tetrafluorborsäure auf 100°C erwärmt. Die orange-braune Lösung wird dann auf 0 - 5°C abgekühlt und mit 10,5 g Natriumnitrit in 25 ml Wasser tropfenweise versetzt. Anschließend gibt man die Reaktionslösung auf 200 ml Eiswasser. Das Produkt wird abgesaugt und mit Tetrahydrofuran gewaschen.
Ausbeute: 60 % 2,4-Dichlor-5-fluorphenyldiazoniumtetrafluoroborat
3,0 g (10,8 mmol) 2,4-Dichlor-5-fluorphenyldiazoniumtetrafluoroborat werden zu einer 60-70°C warmen Lösung von 122 mg Palladiumacetat, 13 ml 4-Methylstyrol und 30 ml Chloroform portionsweise gegeben. Anschließend wird zwei Stunden bei dieser Temperatur nachgerührt. Die Mischung wird im Vakuum eingeengt und chromatographiert (Elutionsmittel: Petrolether/Essigester 20 : 1, Träger SiO₂). Ausbeute: 87 % 2,4-Dichlor-5-fluor-4'-methylstilben
¹H-NMR: 7,08 (d, J = 16 Hz, 1 H, C-H, olefinisch), 7,26 - 7,59 (m, 7 H, C-H, olefinisch, aromatisch).
¹⁹F-NMR (94 MHz): 117,5 (C-F)
MS (EI, 70 eV): 280 (100 %) M⁺

## Patentansprüche

1. Verbindungen der Formel I 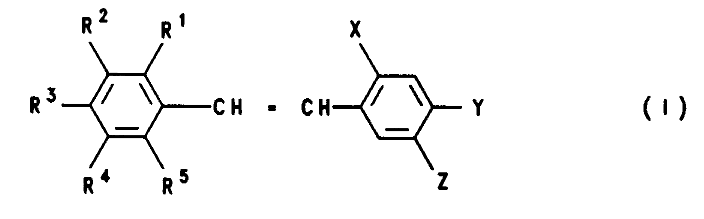 dadurch gekennzeichnet, daß R¹ bis R⁵ unabhängig voneinander Wasserstoff, Alkyl(C₁-C₁₂), Fluor, Chlor, Brom, -OH, -CN, -CHO, -OAlkyl(C₁-C₈), -OPhenyl, -OCOAlkyl(C₁-C₈), -OCOPhenyl, -NH₂, -NHAlkyl(C₁-C₈), -NHPhenyl, -N(Alkyl)₂ (C₁-C₈), -NPhenylAlkyl(C₁-C₈), -N(Phenyl)₂, -NHCOAlkyl(C₁-C₈), -NHCOPhenyl, -NO₂, CN, Alkenyl, Alkinyl, -CF₃ und X, Y, Z ein Halogenatom bedeuten.

2. Verbindungen nach Anspruch 1, dadurch gekennzeichnet, daß R¹ bis R⁵ unabhängig voneinander Wasserstoff, Halogen oder Alkyl(C₁-C₁₂) bedeuten.

3. Verbindungen nach Anspruch 1, dadurch gekennzeichnet, daß R¹ bis R⁵ Wasserstoff, Methyl, Ethyl, Fluor oder Chlor bedeuten.

4. Verbindungen nach mindestens einem der Ansprüche 1 bis 3 dadurch gekennzeichnet, daß X, Y, Z Fluor, Chlor oder Brom, insbesondere Fluor oder Chlor bedeuten.

5. 2,4-Dichlor-5-fluor-stilben, 2,4,5-Trifluorstilben, 2-Chlor-4,5-difluorstilben, 2,4-Dichlor-5-fluor-4'-methylstilben, 2,4-Dichlor-5-fluor-2'-methylstilben, 2,4-Dichlor-5-fluor-3'-methylstilben, 2,4,4'-Trichlor-5-fluorstilben.

6. Verfahren zur Herstellung der Verbindungen nach Anspruch 1 bis 5 dadurch gekennzeichnet, daß man eine Verbindung der Formel II 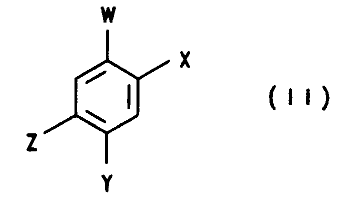 worin X, Y, Z die bereits genannte Bedeutung haben und W ein Iod-, Brom-, Chloratom oder eine Diazoniumgruppe N₂A bedeutet, worin A ein Anion einer Säure mit einem pKₐ-Wert < 7 ist, mit einer Verbindung der allgemeinen Formel (III) 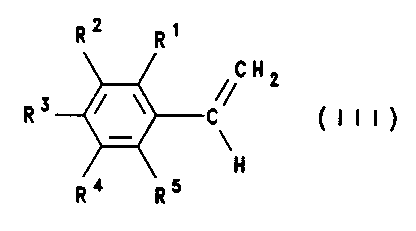 in welcher R¹ bis R⁵ die genannte Bedeutung haben, in Gegenwart katalytischer Mengen eines Palladiumkatalysators, gegebenenfalls in einem Lösungsmittel, gegebenenfalls in Gegenwart einer Base und/oder stabilisierender Liganden und/oder quartärer Ammonium- oder Phosphoniumsalze umsetzt.

7. Verfahren nach Anspruch 6, dadurch gekennzeichnet, daß der Palladiumkatalysator homogen eingesetzt wird.

8. Verfahren nach Anspruch 7, dadurch gekennzeichnet, daß als Palladiumverbindungen Palladium-II-Salze, insbesondere Palladiumacetat, Palladiumdichlorid, Palladiumdibromid, Natriumtetrachloropalladat, Bistriphenylphosphinpalladiumdichlorid, Bisbenzonitrilpalladiumdichlorid, Palladiumsulfat oder deren Gemische eingesetzt werden.

9. Verfahren nach Anspruch 6 oder 7, dadurch gekennzeichnet, daß als Palladiumverbindungen Palladium-(O)-Komplexe eingesetzt werden.

10. Verfahren nach Anspruch 6, dadurch gekennzeichnet, daß der Palladiumkatalysator heterogen eingesetzt wird.

11. Verfahren nach Anspruch 10, dadurch gekennzeichnet, daß der Katalysator auf einem Trägermaterial, insbesondere Aktivkohle, Calciumcarbonat, Bariumsulfat, Bimsstein, Tonerde, Kieselgur, Kieselgel, Graphit, Magnesiumoxid und/oder Aluminiumoxid, bevorzugt Aktivkohle und/oder Aluminiumoxid und/oder Kieselgel eingesetzt wird.

12. Verfahren nach Anspruch 10 oder 11, dadurch gekennzeichnet, daß der Katalysator 0,5 - 20 Gew.-%, insbesondere 1 - 10 Gew.-% Palladium bezogen auf das Trägermaterial enthält.

13. Verfahren nach mindestens einem der Ansprüche 6 bis 12, dadurch gekennzeichnet, daß das Mengenverhältnis Palladium zu Halogenaromat oder Diazoniumverbingung 0,001 bis 20 mol %, insbesondere 0,05 bis 5 mol % beträgt.

14. Verfahren nach mindestens einem der Ansprüche 6 bis 13, dadurch gekennzeichnet, daß man pro Mol Verbindung der Formel (II) 0,5 bis 10 mol, insbesondere 1 bis 5 mol Verbindung der Formel (III) einsetzt.

15. Verfahren nach mindestens einem der Ansprüche 6 bis 14, dadurch gekennzeichnet, daß man als Lösungsmittel ein dipolar aprotisches Lösungsmittel, insbesondere, Dimethylacetamid, Dimethylformamid, N-Methylpyrrolidon, Dimethylsulfoxid, Diglyme oder Tetraglyme, bevorzugt Dimethylacetamid, Dimethylformamid oder N-Methylpyrolidon verwendet.

16. Verfahren nach mindestens einem der Ansprüche 6 bis 14, dadurch gekennzeichnet, daß für die Umsetzung der Diazoniumsalze der Formel (II) als Lösungsmittel Alkohole, Ether oder Nitrile, insbesondere Methanol, Ethanol, Isopropanol, Ethylenglykol, Tetrahydrofuran, Dioxan oder Acetonitril eingesetzt werden.

17. Verfahren nach mindestens einem der Ansprüche 6 bis 16, dadurch gekennzeichnet, daß die Reaktion in einem Mehrphasensystem durchgeführt wird.

18. Verfahren nach mindestens einem der Ansprüche 1 bis 17, dadurch gekennzeichnet, daß als Base Amine, Alkali- und/oder Erdalkalisalze von Carbonsäuren und/oder anorganischen Säuren eingesetzt werden.

19. Verfahren nach mindestens einem der Ansprüche 6 bis 9 und 14 bis 18, dadurch gekennzeichnet, daß als stabilisierende Liganden Phosphine, Phosphite, Nitrile oder Amine, insbesondere Triarylphosphine, Trialkylphosphine, Triethylphosphit, Acetonitril, Benzonitril und/oder Bispyridin zugesetzt werden.

20. Verfahren nach mindestens einem der Ansprüche 6 bis 9 und 14 bis 19, dadurch gekennzeichnet, daß als quartäre Ammonium- oder Phosphoniumsalze Tetra-n-butylammoniumbromid, Tetramethylammoniumchlorid, Tetraphenylammoniumchlorid, Hexadecyltrimethylammoniumbromid und/oder Tetraphenylphosphoniumbromid eingesetzt werden.
